# EUROPÄISCHE PATENTSCHRIFT

(11) **EP 4 262 615 B1**
(45) Veröffentlichungstag und Bekanntmachung des Hinweises auf die Patenterteilung: **29.01.2025**
(21) Anmeldenummer: 21816470.5
(22) Anmeldetag: 30.11.2021
(51) Int. Cl.: A61C 8/00, A61C 13/235, A61C 19/04

(54) **DENTALES BEFESTIGUNGSSYSTEM**
DENTAL ATTACHMENT SYSTEM
SYSTÈME DENTAIRE DE FIXATION

(30) Priorität: 16.12.2020 EP 20214669
(43) Veröffentlichungstag der Anmeldung: 25.10.2023
(73) Patentinhaber: Ivoclar Vivadent AG, 9494 Schaan (LI)
(72) Erfinder: GROSSE-HONEBRINK, Alexander, 9463 Oberriet (CH); MUELLER, Frank, 6800 Feldkirch (AT); SCHUELKE, Andreas, 9472 Grabs (CH); SENTI, Theresa Sujata Maria, 9497 Triesenberg (LI)
(74) Vertreter: Baldus, Oliver
(86) Internationale Anmeldenummer: PCT/EP2021/083507
(87) Internationale Veröffentlichungsnummer: WO 2022/128437

(56) Entgegenhaltungen:
- US-A- 4 209 905
- US-A- 4 508 507
- US-A- 5 337 033
- US-A1- 2003 118 969
- US-A1- 2007 005 042
- US-B1- 6 171 107

## Beschreibung

Die vorliegende Erfindung betrifft ein dentales Befestigungssystem. Ein Dentalobjekt und ein Verfahren zum Befestigen eines Dentalobjekts werden auch offenbart.

Heutige intraorale Befestigungsmöglichkeiten werden beispielsweise durch mechanische Klemmen oder mittels einer chemischen Adhäsion realisiert. Klemmverfahren verwenden beispielsweise Klemmen, die um ein oder mehrere Zähne gelegt werden, dentale Aligner, die einen ganzen oder einen Teil eines Zahnbogens umfassen oder Bänder, die um ein oder mehrere Zähne gelegt werden. Bei den Klemmverfahren kann jedoch die Zahnsubstanz beschädigt werden oder durch den ausgeübten Druck Zähne verschoben werden.

Die Adhäsion wird mittels eines Klebemittels oder Zements erreicht. Eine Befestigung mittels einer Adhäsion ist jedoch dauerhaft und muss von einem Zahnarzt entfernt werden. Dies kann zu Problemen für Patienten führen, da diese ein befestigtes Dentalobjekt bei der Mundhygiene nicht selbst entfernen können. Eine Haftcreme ermöglicht hingegen nur eine schwache Befestigung, so dass ein Tragen eines verschluckbaren Dentalobjekts über Nacht nicht möglich ist.

Die Druckschrift US 4,209,905 A betrifft die Verankerung von künstlichen Zahnprothesen durch Verwendung von Magnetelementen.

Die Druckschrift US 2003/118969 A1 betrifft eine magnetische Zahnhalterung, die einen in einer Zahnwurzel eingebetteten Halter und eine magnetische Anordnung umfasst, die durch magnetische Anziehungskraft frei an dem Halter befestigt oder von diesem abgenommen wird.

Die Druckschrift US 5,337,033 A betrifft eine Permanentmagnetanordnung zum stabilen Fixieren einer Zahnprothese unter Verwendung einer magnetischen Anziehungskraft eines Permanentmagneten.

Die Druckschrift US 4,508,507 A betrifft eine verbesserte magnetische Anordnung zum Halten von vollständigen und partiellen Zahnprothesen.

Die Druckschrift US 6,171,107 B1 betrifft ein Verfahren, Verbindungen und Vorrichtungen zum Aneinanderkleben von Gegenständen, so dass sie durch Anlegen von Magnetfeldern entfernt werden.

Die Druckschrift D6 - US 2007/005042 A1 betrifft ein Verfahren und Vorrichtungen zum Verabreichen von Arzneimitteln oder therapeutischen Mitteln.

Es ist die technische Aufgabe der vorliegenden Erfindung, eine lösbare und reversible Befestigung eines Dentalobjekts im Intraoralraum zu ermöglichen.

Diese Aufgabe wird durch den Gegenstand gemäß Anspruch 1 gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein dentales Befestigungssystem gelöst, mit einem ersten Magnetelement zur Befestigung in einer Mundhöhle, das auf einer Kappe befestigt ist, die auf einem Zahn oder Zähnen aufsetzbar ist; und einem Dentalobjekt mit einem zweiten Magnetelement zur Befestigung an dem ersten Magnetelement. Das erste Magnetelement kann beispielsweise an einem oder mehreren Zähnen, einer Spange, einer Bissschiene, einem Abutment, einem Zahnstumpf, einer Prothese, oder einer Teilprothese befestigt werden.

Mittels einer lösbaren Magnethalterung durch die beiden Magnetelemente kann das Dentalobjekt jederzeit von dem Zahn entfernt werden. Das Magnetelement auf dem Zahn kann jedoch auch im Mund verbleiben, bis dieses vom Zahnarzt oder vom Patienten selbst entfernt wird. Da das eine Magnetelement auf dem zuvor gereinigten Zahnschmelz aufgetragen wird, entsteht kein Problem bei einer Mundhygiene. Durch das Befestigungssystem wird daher der technische Vorteil erreicht, dass nichtprothetische oder nicht-orthodontische Dentalobjekte im Intraoralbereich reversibel befestigt werden können.

Die Kappe ist beispielsweise eine Kunststoffkappe, die der Form des Zahns angepasst ist und von oben auf den Zahn gesetzt wird. Dadurch wird beispielsweise der technische Vorteil erreicht, dass das Magnetelement auf einfache Weise reversibel an dem Zahn befestigt werden kann.

In einer technisch vorteilhaften Ausführungsform des Befestigungssystems wird das erste Magnetelement durch einen Magnetlack, ein Magnetadhäsiv oder einen Magnetzement gebildet, der auf einen Zahn oder Zähne auftragbar ist. Dadurch wird beispielsweise der technische Vorteil erreicht, dass das erste Magnetelement auf eine schnelle und einfache Weise auf jedem beliebigen Zahn angeordnet werden kann.

In einer weiteren technisch vorteilhaften Ausführungsform des Befestigungssystems umfasst der Magnetlack ferrimagnetische, ferromagnetische oder dauermagnetische Partikel. Die Partikel können eine Größe im Nanometerbereich oder Mikrometerbereich aufweisen. Dadurch wird beispielsweise der technische Vorteil erreicht, dass eine starke Anziehungskraft zwischen den Magnetelementen erreicht werden kann.

In einer weiteren technisch vorteilhaften Ausführungsform des Befestigungssystems ist das erste Magnetelement durch einen Dauermagneten oder einen Ferromagneten gebildet, der auf den Zahn oder Zähnen befestigbar ist. Die Befestigung kann mittels Befestigungsmethode einem Lack, einem Adhäsiv oder einem Zement erfolgen. Dadurch wird beispielsweise der technische Vorteil erreicht, dass eine vorbestimmte Anziehungskraft und gute Haftung zwischen den Magnetelementen erreicht wird.

In einer weiteren technisch vorteilhaften Ausführungsform des Befestigungssystems umfasst das zweite Magnetelement einen Dauermagneten, einen Elektromagnet und/oder ein Ferromagnet. Das zweite Magnetelement kann auch durch einen Magnetlack gebildet sein. In diesem Fall kann das erste Magnetelement ein Dauermagnet sein. Dadurch wird beispielsweise der technische Vorteil erreicht, dass eine gute Anziehungskraft zu dem ersten Magnetelement erreicht wird.

In einer weiteren technisch vorteilhaften Ausführungsform des Befestigungssystems weist das zweite Magnetelement oder das Dentalobjekt eine Oberflächenkrümmung auf, die einem Zahn angepasst ist. Die Oberflächenkrümmung des Magnetelements oder des Dentalobjekts kann dabei ungefähr der Krümmung des Zahnes folgen. Dadurch wird beispielsweise der technische Vorteil erreicht, dass eine Verschiebung des Dentalobjekts gegenüber dem Zahn zusätzlich erschwert werden kann.

In einer weiteren technisch vorteilhaften Ausführungsform des Befestigungssystems umfasst das zweite Magnetelement eine Schicht, die durch die Anziehungskraft zwischen den beiden Magnetelementen deformierbar ist. Die Schicht kann beispielsweise eine Schaumstoffschicht oder eine Moosgummischicht sein, die durch die Anziehungskraft der Magnetelemente eingedrückt wird. Dadurch wird beispielsweise der technische Vorteil erreicht, dass das Dentalobjekt zusätzlich an dem Zahn stabilisiert wird.

In einer weiteren technisch vorteilhaften Ausführungsform des Befestigungssystems umfasst das erste Magnetelement eine erste Kontaktfläche zu dem zweiten Magnetelement und/oder das zweite Magnetelement eine zweite Kontaktfläche zu dem ersten Magnetelement. Die erste und die zweite Kontaktfläche können beispielsweise durch ebene Flächen gebildet sein. Dadurch wird beispielsweise der technische Vorteil erreicht, dass der Abstand des Zahns und des Dentalobjekts durch die Kontaktflächen gegeben ist und das Dentalobjekt stabilisiert wird, so dass ein Wackeln des Dentalobjekts aufgrund der Anziehung zwischen beiden Kontaktflächen verhindert werden kann.

In einer weiteren technisch vorteilhaften Ausführungsform des Befestigungssystems sind in der ersten und der zweiten Kontaktfläche räumliche Strukturen gebildet, die einen eine vorgegebene Orientierung und/oder einen Formschluss zwischen dem ersten und dem zweiten Magnetelement bewirken. Durch die Strukturen wird beispielsweise der technische Vorteil erreicht, dass eine Drehung zwischen den beiden Magnetelementen und/oder eine Verschiebung zwischen den beiden Magnetelementen verhindert werden kann.

In einer weiteren technisch vorteilhaften Ausführungsform des Befestigungssystems umfasst das Dentalobjekt einen Dentalsensor, ein Elektronikbauteil, eine Batterie und/oder ein Gehäuse. Dadurch wird beispielsweise der technische Vorteil erreicht, dass physikalische Eigenschaften an dem Zahn elektronisch bestimmt werden können.

In einer weiteren technisch vorteilhaften Ausführungsform des Befestigungssystems umfasst das erste Magnetelement, der

Sensor und/oder das Sensorgehäuse ein oder mehrere Kanäle zum Führen von Speichel zu einer Sensoreinheit. Dadurch wird beispielsweise ebenfalls der technische Vorteil erreicht, dass Eigenschaften des Speichels durch das Dentalobjekt analysiert werden können.

In einer weiteren technisch vorteilhaften Ausführungsform des Befestigungssystems ist das zweite Magnetelement im Inneren des Dentalobjekts oder außen am Dentalobjekt angeordnet ist und/oder das Dentalobjekt umfasst ein Gehäuse, dass ein ferromagnetisches, ferrimagnetisches oder dauermagnetisches Material oder einen Elektromagneten umfasst. Dadurch wird beispielsweise der technische Vorteil erreicht, dass das Magnetelement nicht nach außen ragt und vor Beschädigungen oder Feuchtigkeit geschützt ist. Zudem wird beispielsweise der technische Vorteil erreicht, dass das Magnetelement durch das Gehäuse gebildet wird und in diesem integriert ist. Das Gehäuse kann auch eine Batterie oder einen Akkumulator umfassen, die zu einer Stromversorgung verwendet wird und zudem als Magnetelement dient.

Gemäß einem zweiten Aspekt, der aber nicht Teil der beanspruchten Erfindung ist, wird die technische Aufgabe durch ein Verfahren zum Befestigen eines Dentalobjekts gelöst, mit den Schritten eines Befestigens eines ersten Magnetelements in einer Mundhöhle, das auf einer Kappe befestigt ist, die auf einem Zahn oder Zähnen aufsetzbar ist; und eines Befestigens eines Dentalobjekt mit einem zweiten Magnetelement an dem ersten Magnetelement. Durch das Verfahren werden die gleichen technischem Vorteile wie durch Befestigungssystem nach dem ersten Aspekt gelöst.

Ausführungsbeispiele der Erfindung sind in den Zeichnungen dargestellt und werden im Folgenden näher beschrieben.

Es zeigen:
- Fig. 1: eine schematische Ansicht eines dentalen Befestigungssystems;
- Fig. 2: eine weitere schematische Ansicht eines dentalen Befestigungssystems;
- Fig. 3: eine Struktur zum Herstellen eines Formschlusses zwischen den beiden Magnetelementen;
- Fig. 4: eine schematische Ansicht eines Magnetelements mit einer deformierbaren Schicht;
- Fig. 5: eine schematische Ansicht von Kanälen zum Führen von Speichel; und
- Fig. 6: ein Blockdiagramm des Verfahrens zum Befestigen eines Dentalobjekts, das aber nicht Teil der beanspruchten Erfindung ist.

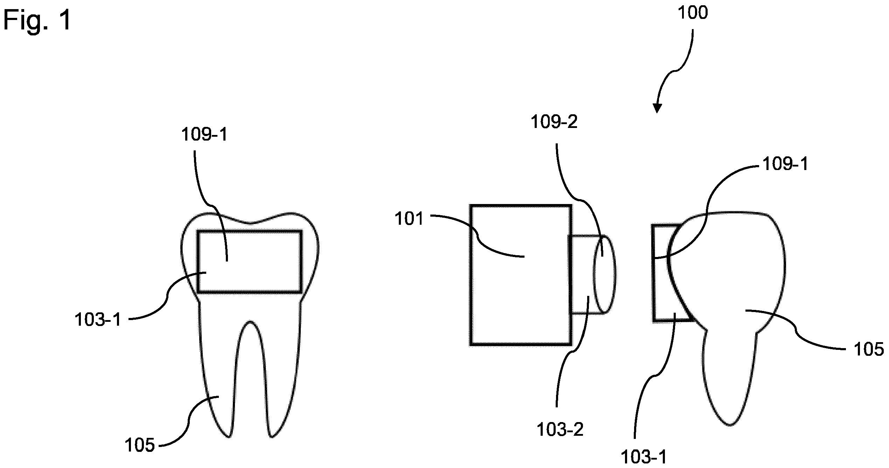

Fig. 1 zeigt eine schematische Ansicht eines dentalen Befestigungssystems 100 für ein Dentalobjekt 101 in einer Mundhöhle. Das Dentalobjekt 101 kann jedes räumliche Objekt sein, das in einer Mundhöhle des Patienten befestigt werden soll. Das Dentalobjekt 101 weist eine Geometrie auf, die eine Befestigung im Inneren der Mundhöhle zulässt. Die Befestigung des Dentalobjekts 101 kann an einem oder mehreren Zähnen, einer Spange, einer Bissschiene, einem Abutment, einem Zahnstumpf, einer Prothese, oder einer Teilprothese erfolgen. Als erstes Magnetelement 103-1 dient ein Magnetlack, der manuell auf dem Zahn 105 im ausgewählten Intraoralbereich aufgetragen wird. Dieser Magnetlack umfasst beispielsweise gesundheitsverträgliche, geschliffene und/oder ferromagnetische oder dauermagnetische Partikel mit Größen im Nanometerbereich oder Mikrometerbereich, die in diesen eingemischt sind. Beispielsweise kann der Magnetlack Eisenpartikel, Neodym-Partikel (Neodym-Eisen-Bor - NdFeB) oder Kobalt-Partikel (Aluminium-Nickel-Cobalt - AlNiCo) umfassen, die zur Erzeugung der magnetischen Eigenschaften des Magnetelements 103-1 dienen.

Der Magnetlack kann auf den Zahn 105 aufgestrichen werden. Der Magnetlack kann ein pastöses und/oder lichthärtendes Material mit magnetischen Füllstoffen sein. Dadurch ergibt sich der Vorteil, dass der Magnetlack flach aufgetragen werden oder plastisch ausgeformt werden kann. Die Ausformung kann mittels einer transparenten Gießschablone, beispielsweise aus Silikon, erfolgen. Die Gießschablone kann eine räumliche Struktur umfassen, die ein räumliches Formschlusselement in dem gehärteten Magnetlack erzeugt, wie beispielsweise eine Dreiecksstruktur.

Der Magnetlack kann beispielsweise auf Basis geeigneter Polymere hergestellt sein, wie beispielsweise Copolymere aus t-Butylacrylat, Ethylacrylat, Methacrylsäure; Copolymere aus Ethylacrylat und Methacrylsäure, Copolymere aus N-tert.-Butylacrylamid, Ethylacrylat, Acrylsäure; Copolymere aus Vinylacetat, Crotonsäure und gegebenenfalls weitere Vinylester, anionische Polysiloxane, z.B. carboxyfunktionelle, t-Butylacrylat, Methacrylsäure. Beispiele für anionische Polymere sind weiterhin Vinylacetat/Crotonsäure-Copolymere.

Als erstes Magnetelement 103-1 kann auch ein Magnetzement dienen, der manuell auf dem Zahn 105 im ausgewählten Intraoralbereich aufgetragen wird. Der Magnetzement kann beispielsweise auf Basis geeigneter Materialien hergestellt werden, wie beispielsweise mono- oder polyfunktionelle Methacrylate, wie Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-Dimethacrylat, Bis-GMA (ein Additionsprodukt aus Methacrylsäure und Bisphenol-Adiglycidylether), ethoxy- oder propoxyliertes Bisphenol-A-Dimethacrylat.

Magnetadhäsive können sein: HEMA, Bis-GMA, D3MA, MDP, Ethanol, Wasser, methacrylat-modifizierte Polyacrylsäure, Siliciumdioxid, Campherchinon, Ethyl p-dimethylaminobenzoat, 2-dimethylaminoethyl methacrylat.

Das Dentalobjekt 101 umfasst ein zweites Magnetelement 103-2, das zur Befestigung an dem ersten Magnetelement 103-1 dient. Das Magnetelement 103-2 des Dentalobjekts 101 wirkt als Gegenpol zu dem Magnetelement 103-1 auf dem Zahn 105. Dadurch entsteht eine Anziehungskraft zwischen den beiden Magnetelementen 103-1 und 103-2, die zur Befestigung des Dentalobjektes 101 dient.

Nach dem Auftragen des Magnetlacks auf den Zahnschmelz des Zahns 105 kann daher ein Dentalobjekt 101, das mit dem gegenüberliegenden Magnetelement 103-2 ausgestattet ist, auf der modifizierten Zahnoberfläche befestigt werden. Das Magnetelement 103-2 des Dentalobjekts 101 kann beispielsweise durch einen Dauermagnet (Permanentmagnet), einen Ferromagnet oder einen Elektromagneten gebildet sein. Der Elektromagnet des Dentalobjekts 100 kann wahlweise von einer Elektronik aktiviert werden. Der Permanentmagnet kann derart ausgestaltet sein, dass dieser ein mechanisch schaltbares Magnetfeld erzeugt. Beispielsweise können Einzelmagnete des Permanentmagneten in einer ersten räumlichen Anordnung ein wirksames Magnetfeld für die Befestigung erzeugen und in einer zweiten Anordnung das Magnetfeld für die Befestigung aufheben. Diese Wirkung entsteht aus dem Superpositionsprinzip der Magnetfelder.

Das Magnetelement 103-2 kann beispielsweise durch einen ferromagnetischen oder dauermagnetischen räumlichen Körper gebildet sein. Als ferromagnetisches Material kann beispielsweise Eisen verwendet werden. Als dauermagnetisches Material kann beispielsweise Neodym verwendet werden. Dieses kann mit verschiedenen biokompatiblen Materialen wie Gold beschichtet werden. Im Allgemeinen können aber auch alle anderen magnetischen Materialien verwendet werden, durch die eine ausreichende Anziehungskraft hergestellt werden kann.

Das Magnetelement 103-2 wirkt als Gegenpol zu dem Magnetelement 103-1 auf dem Zahn 105. Durch den ferromagnetischen oder dauermagnetischen Magnetlack auf dem Zahn 105 wird ebenfalls ein Gegenpol zu dem Magnetelement 103-2 im Inneren des Dentalobjekts 101 oder auf dem Dentalobjekt 101 gebildet.

Das zu befestigende Dentalobjekt 101 kann beispielsweise ein Dentalsensor, ein Elektronikbauteil, eine Batterie, und/oder ein Gehäuse für verschiedene Bestandteile sein. Der Dentalsensor ist ein Dentalobjekt 101, das in der Mundhöhle angeordnet werden kann und eine Elektronik zur autonomen Erfassung unterschiedlicher Messwerte umfasst. Beispielsweise kann der Dentalsensor eine Elektronik zur Erfassung eines pH-Wertes in der Mundhöhle umfassen.

Das Gehäuse kann aus einem Polymermaterial, Metall oder einem kohlenstofffaserverstärkten Kunststoff (Karbonmaterial) gefertigt sein. Zudem kann das Gehäuse auch dauermagnetische oder ferromagnetische Partikel umfassen, so dass das zweite Magnetelement 103-2 durch das Gehäuse gebildet wird und in diesem intergiert ist. Daneben kann es vorteilhaft sein, dass zweite Magnetelement 103-2 im Inneren des Gehäuses anzuordnen, da dieses dann vor Korrosion geschützt ist. Das Dentalobjekt 101 weist eine Größe auf, dass dieses im Mundraum eines Patienten angeordnet werden kann. Das Gehäuse kann in einem dreidimensionalen Druckverfahren erzeugt werden, bei dem ein Material mit magnetischen Partikeln verwendet wird.

Der Magnetlack des ersten Magnetelements 103-1 kann solange im Mundraum verbleiben, bis dieser vom Zahnarzt oder vom Patienten selbst entfernt wird. Da der Magnetlack jedoch nur auf dem zuvor gereinigten Zahnschmelz aufgetragen wird, entsteht nach dem Abnehmen des Dentalobjekts 101 kein Problem bei der Mundhygiene.

Durch die Magnetfelder, die durch die Magnetelemente 103-1 und 103-2 ausgeübt werden, kann das Dentalobjekt 101 in der Mundhöhle reversibel befestigt werden. Im Gegensatz zu einem Klemmverfahren hat der Magnetlack keinen Einfluss auf die Zahnstellung, so dass keine ungewollte Verschiebung des Zahns 105 stattfindet und aufgrund der Magnethalterung kann das Dentalobjekt 101 jederzeit entfernt werden.

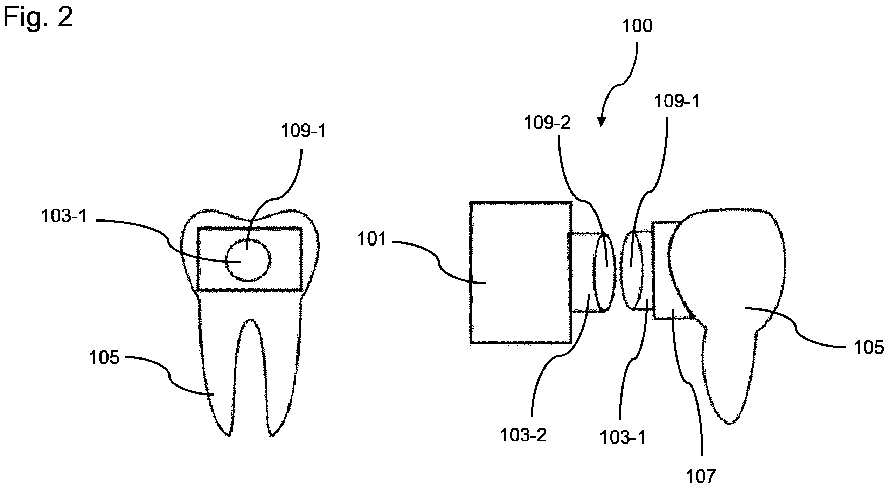

Fig. 2 zeigt eine weitere schematische Ansicht eines dentalen Befestigungssystems 100. In dieser Ausführungsform wird ein körperlicher Dauermagnet oder Ferromagnet als erstes Magnetelement 103-1 mittels eines dentalen Adhäsives, Lackes oder Dentalzements auf dem Zahnschmelz des Zahns 105 befestigt.

Im Allgemeinen kann das erste Magnetelement 103-1 auch auf andere geeignete Art und Weise an dem Zahn 105 befestigt werden. Beispielsweise kann das Magnetelement 103-1 auch auf einer Kappe befestigt sein, die auf den Zahn 105 aufsetzbar ist. Die Kappe ist beispielsweise eine Kunststoffkappe, die der Form des Zahns 105 angepasst ist und von oben auf den Zahn 105 gesetzt wird. Durch die Kappe kann das Magnetelement auf einfache Weise reversibel an dem Zahn 105 befestigt werden.

Dieses erste Magnetelement 103-1 wirkt ebenfalls als Gegenpol zu dem zweiten Magnetelement 103-2 auf dem Dentalobjekt 101, das gleichsam durch einen körperlichen Dauermagneten oder Ferromagneten oder einen Elektromagneten gebildet wird.

Im Gegensatz zu einem Klemmverfahren hat ein aufzementierter Magnet als erstes Magnetelement 103-1 auf dem Zahn 105 ebenfalls keinen Einfluss auf die Zahnstellung und ermöglicht ein reversibles Entfernen des Dentalobjektes 101.

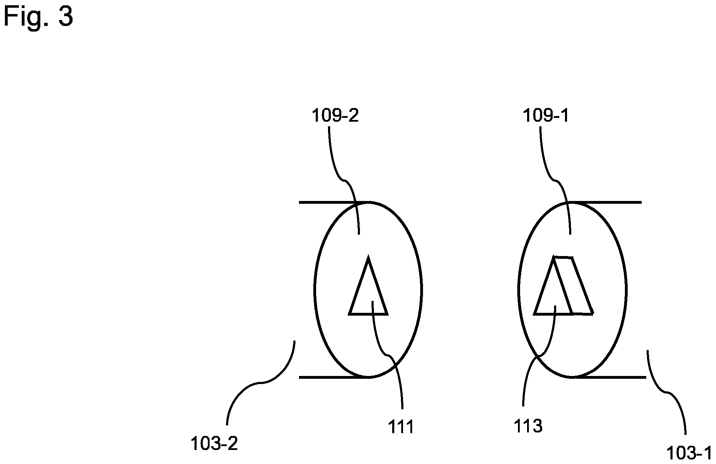

Fig. 3 zeigt eine Struktur zum Herstellen einer Orientierung und eines Formschlusses zwischen den beiden Magnetelementen 103-1 und 103-2. Das erste Magnetelement 103-1 umfasst eine erste Kontaktfläche 109-1 zu dem zweiten Magnetelement 103-2 und das zweite Magnetelement 103-2 umfasst eine zweite Kontaktfläche 109-2 zu dem ersten Magnetelement 103-2. In der ersten und der zweiten Kontaktfläche 109-1 und 109-2 sind jeweils räumliche Strukturen gebildet, die einen Formschluss zwischen dem ersten und dem zweiten Magnetelement 103-1, 103-2 bewirken. Durch diesen Formschluss kann das Dentalobjekt 101 in seiner vorgesehenen Position gehalten werden. Eine Drehung und Verschiebung des Dentalobjektes 101 kann dadurch verhindert werden.

In diesem Fall ist auf der zweiten Kontaktfläche 109-2 eine Aussparung 111 mit einem Dreiecksprofil gebildet. In der gegenüberliegenden Kontaktfläche 109-1 ist ein entsprechender Vorsprung 113 gebildet, der aus der ersten Kontaktfläche 109-1 herausragt. Der Vorsprung 113 wird durch die Anziehungskraft zwischen den beiden Magnetelementen 109-1 und 109-2 in der Aussparung 111 gehalten und verhindert eine gegenseitige Bewegung zwischen den beiden Magnetelementen 103-1 und 103-2.

Im Allgemeinen können jedoch auch andere räumliche, ineinandergreifende Strukturen zur Orientierung und für den Formschluss zwischen den beiden Magnetelementen 103-1 und 103-2 verwendet werden.

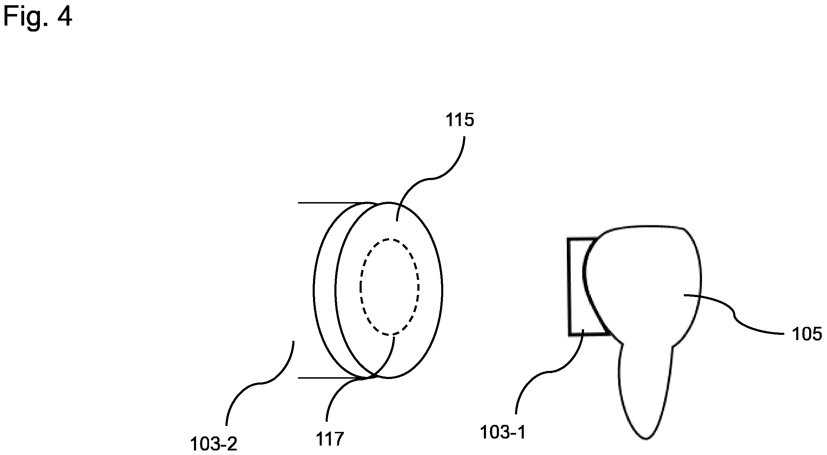

Fig. 4 zeigt eine schematische Ansicht eines Magnetelements 103-2 mit einer deformierbaren Schicht 115. Die Schicht ist aus einem weichelastischen Material, wie beispielsweise einem Schaumstoff, einem Moosgummi oder einer elastomeren Masse, wie Silikon gebildet. Durch die Anziehungskraft zwischen den beiden Magnetelementen 103-1 und 103-2 wird die Schicht 115 im Bereich 117 deformiert. Dadurch kann eine seitliche Verschiebung oder ein gegenseitiges Verrutschen der Magnetelemente 103-1 und 103-2 verhindert werden.

Zudem kann das Magnetelement 103-2 oder das Dentalobjekt 101 zu diesem Zweck eine feste Oberflächenkrümmung aufweisen, die zumindest näherungsweise dem Zahn 105 entspricht, an dem das Dentalobjekt 101 gehalten werden soll. Die Oberflächenkrümmung kann beispielsweise durch eine Ausbuchtung in der Kontaktfläche 109-2 erzeugt werden, die der Form des Zahnes 105 entspricht.

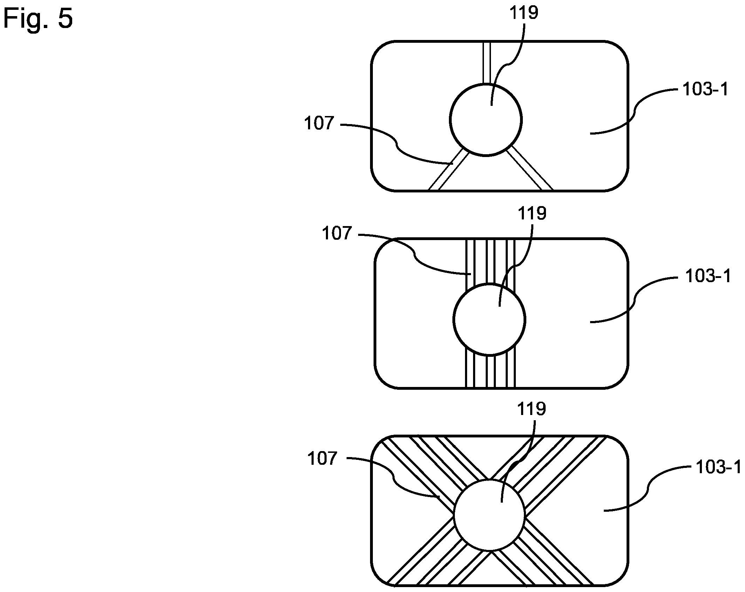

Fig. 5 zeigt eine schematische Ansicht von Kanälen zum Führen von Speichel. Das erste Magnetelement 103-1, der Sensor und/oder das Sensorgehäuse können ein oder mehrere Kanäle 107 zum Führen von Speichel zu einer Sensoreinheit umfassen. In diesem Fall können Eigenschaften des Speichels durch in der Durchgangsöffnung 119 analysiert werden. Die Kanäle 107 dienen für einen Flüssigkeitsabfluss oder eine Luftzuführung. Die Durchgangsöffnung 119 dient zum Ermöglichen einer Messung durch eine Sensoreinheit und bildet einen Messbereich für die Sensoreinheit.

Die Kanäle 107 sind in dem Haftbereich 103 durch Vertiefungen gebildet und dienen dazu Flüssigkeit (Speichel) zu der Sensoreinheit hinzuführen oder eine Ventilation des Messbereichs zu ermöglichen. Die Kanäle 107 können in horizontaler, diagonaler oder vertikaler Richtung angeordnet sein und U- oder V-förmig gebildet sein.

Der Speichelfluss kann neben der Messungsmöglichkeit auch einen angenehmeren Tragekomfort erzeugen. Zudem verursachen die Kanäle 107 eine Materialeinsparung und leichtere Wiederentfernbarkeit. Die Kanäle 107 für den Speichel können in dem ersten Magnetelement 103-1, dem Sensor und/oder dem Sensorgehäuse vorgesehen sein.

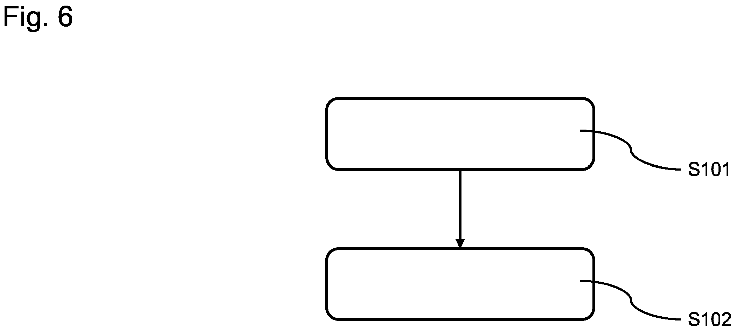

Fig. 6 zeigt ein Blockdiagramm des Verfahrens zum Befestigen des Dentalobjekts 101, das aber nicht Teil der beanspruchten Erfindung ist. . In einem ersten Schritt S101 wird das erste Magnetelement 103-1 an dem Zahn 105 befestigt. Im zweiten Schritt S102 wird das Dentalobjekt 101 mit dem zweiten Magnetelement 103-2 an dem ersten Magnetelement 103-1 befestigt.

Das Verfahren verwendet Magnetfelder, um das Dentalobjekt 101 in der Mundhöhle zu befestigen. Diese Magnetfelder können im Allgemeinen auf unterschiedliche Weise erzeugt werden. Eine Magnetbefestigung mittels der beiden Magnetelemente 103-1 und 103-2 kann in Scherrichtung und in Zugrichtung deutlich stärker als eine Befestigung mittels Haftcreme sein.

Mittels einer Magnethalterung kann das Dentalobjekt 101 jederzeit entfernt werden. Der Magnetlack, Magnetadhäsiv, Magnetzement oder der geklebte oder zementierte Dauermagnet können im Mundraum verbleiben, bis diese vom Zahnarzt oder vom Patienten selbst entfernt werden. Da der Magnetlack, das Magnetadhäsiv oder der Magnetzement jedoch nur auf dem zuvor gereinigten Zahnschmelz des Zahns 105 aufgebracht werden, entsteht kein Problem mit der Mundhygiene.

Es entsteht eine starke, einfach reversible Befestigung des Dentalobjekts 101 mit dem Zahn 105 im Intraoralraum. Dabei ist besonders die Reversibilität vorteilhaft, da ein Patient nach einer manuellen Entfernung des Dentalobjekts 101 seine gewohnte Mundhygiene ausüben kann und keine Sekundärerkrankungen durch das Dentalobjekt 101 ausgelöst werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Befestigungssystem
- 101: Dentalobjekt
- 103: Magnetelement
- 105: Zahn
- 107: Kanal
- 109: Kontaktfläche
- 111: Aussparung
- 113: Vorsprung
- 115: Schicht
- 117: Bereich
- 119: Durchgangsöffnung

## Patentansprüche

1. Dentales Befestigungssystem (100), mit:
- einem ersten Magnetelement (103-1) zur Befestigung in einer Mundhöhle, das auf einer Kappe befestigt ist, die auf einem Zahn (105) oder Zähnen aufsetzbar ist; und
- einem Dentalobjekt (101) mit einem zweiten Magnetelement (103-2) zur Befestigung an dem ersten Magnetelement (103-1).

2. Befestigungssystem (100) nach Anspruch 1, wobei das erste Magnetelement (103-1) durch einen Magnetlack, ein Magnetadhäsiv oder einen Magnetzement gebildet wird, der auf einen Zahn (105) oder Zähne auftragbar ist.

3. Befestigungssystem (100) nach Anspruch 2, wobei der Magnetlack ferrimagnetische, ferromagnetische oder dauermagnetische Partikel umfasst.

4. Befestigungssystem (100) nach einem der vorangehenden Ansprüche, wobei das erste Magnetelement (103-1) durch einen Dauermagneten oder einen Ferromagneten gebildet ist, der auf den Zahn (105) oder Zähnen befestigbar ist.

5. Befestigungssystem (100) nach einem der vorangehenden Ansprüche, wobei das zweite Magnetelement (103-2) einen Dauermagneten, einen Elektromagneten und/oder einen Ferromagneten umfasst.

6. Befestigungssystem (100) nach einem der vorangehenden Ansprüche, wobei das zweite Magnetelement (103-2) oder das Dentalobjekt (101) eine Oberflächenkrümmung aufweist, die einem Zahn (105) angepasst ist.

7. Befestigungssystem (100) nach einem der vorangehenden Ansprüche, wobei das zweite Magnetelement (103-2) eine Schicht umfasst, die durch die Anziehungskraft zwischen den beiden Magnetelementen (103-1, 103-2) deformierbar ist.

8. Befestigungssystem (100) nach einem der vorangehenden Ansprüche, wobei das erste Magnetelement (103-1) eine erste Kontaktfläche (109-1) zu dem zweiten Magnetelement (103-2) umfasst und/oder das zweite Magnetelement (103-2) eine zweite Kontaktfläche (109-2) zu dem ersten Magnetelement (103-1) umfasst.

9. Befestigungssystem (100) nach einem der vorangehenden Ansprüche, wobei in der ersten und der zweiten Kontaktfläche (109-1, 109-2) räumliche Strukturen gebildet sind, die eine vorgegebene Orientierung und/oder einen Formschluss zwischen dem ersten und dem zweiten Magnetelement (103-1, 103-2) bewirken.

10. Befestigungssystem (100) nach einem der vorangehenden Ansprüche, wobei das Dentalobjekt (101) einen Dentalsensor, ein Elektronikbauteil, eine Batterie und/oder ein Gehäuse umfasst.

11. Befestigungssystem (100) nach einem der vorangehenden Ansprüche, wobei das erste Magnetelement (103-1), der Sensor und/oder das Sensorgehäuse ein oder mehrere Kanäle zum Führen von Speichel zu einer Sensoreinheit umfasst.

12. Befestigungssystem (100) nach einem der vorangehenden Ansprüche, wobei das zweite Magnetelement (103-2) im Inneren des Dentalobjekts (101) oder außen am Dentalobjekt (101) angeordnet ist und/oder das Dentalobjekt (101) ein Gehäuse umfasst, dass ein ferromagnetisches, ferrimagnetisches oder dauermagnetisches Material oder einen Elektromagneten umfasst.

## Claims

1. A dental attachment system (100), comprising:
- a first magnetic element (103-1) for attachment in an oral cavity which is attached to a cap which can be placed on a tooth (105) or teeth; and
- a dental object (101) having a second magnetic element (103-2) for attachment to the first magnetic element (103-1).

2. The attachment system (100) according to claim 1, wherein the first magnetic element (103-1) is formed by a magnetic varnish, a magnetic adhesive, or a magnetic cement that is applicable to a tooth (105) or teeth.

3. The attachment system (100) according to claim 2, wherein the magnetic varnish comprises ferrimagnetic, ferromagnetic, or permanent magnetic particles.

4. The attachment system (100) according to any one of the preceding claims, wherein the first magnetic element (103-1) is formed by a permanent magnet or a ferromagnet attachable to the tooth (105) or teeth.

5. The attachment system (100) according to any one of the preceding claims, wherein the second magnetic element (103-2) comprises a permanent magnet, an electromagnet and/or a ferromagnet.

6. The attachment system (100) according to any one of the preceding claims, wherein the second magnetic element (103-2) or the dental object (101) has a surface curvature adapted to a tooth (105).

7. The attachment system (100) according to any one of the preceding claims, wherein the second magnetic element (103-2) comprises a layer deformable by the attractive force between the two magnetic elements (103-1, 103-2).

8. The attachment system (100) according to any one of the preceding claims, wherein the first magnetic element (103-1) comprises a first contact surface (109-1) to the second magnetic element (103-2) and/or the second magnetic element (103-2) comprises a second contact surface (109-2) to the first magnetic element (103-1).

9. The attachment system (100) according to any one of the preceding claims, wherein spatial structures are formed in the first and second contact surfaces (109-1, 109-2) to provide a predetermined orientation and/or positive engagement between the first and second magnetic elements (103-1, 103-2).

10. The attachment system (100) according to any one of the preceding claims, wherein the dental object (101) comprises a dental sensor, an electronic component, a battery, and/or a housing.

11. The attachment system (100) according to any one of the preceding claims, wherein the first magnetic element (103-1), the sensor and/or the sensor housing comprises one or more channels for guiding saliva to a sensor unit.

12. The attachment system (100) according to any one of the preceding claims, wherein the second magnetic element (103-2) is arranged inside the dental object (101) or outside the dental object (101) and/or the dental object (101) comprises a housing comprising a ferromagnetic, ferrimagnetic or permanent magnetic material or an electromagnet.

## Revendications

1. Système de fixation dentaire (100), avec :
- un premier élément magnétique (103-1) destiné à être fixé dans une cavité buccale, qui est fixé à un capuchon qui peut être placé sur une dent (105) ou des dents ; et
- un objet dentaire (101) avec un deuxième élément magnétique (103-2) destiné à être fixé au premier élément magnétique (103-1).

2. Système de fixation (100) selon la revendication 1, où le premier élément magnétique (103-1) est formé par un vernis magnétique, un adhésif magnétique ou un ciment magnétique qui peut être appliqué sur une dent (105) ou des dents.

3. Système de fixation (100) selon la revendication 2,
où le vernis magnétique comprend des particules ferrimagnétiques, ferromagnétiques ou magnétiques permanentes.

4. Système de fixation (100) selon l'une des revendications précédentes, où le premier élément magnétique (103-1) est formé par un aimant permanent ou un ferromagnétique qui peut être fixé sur la dent (105) ou les dents.

5. Système de fixation (100) selon l'une des revendications précédentes, où le second élément magnétique (103-2) comprend un aimant permanent, un électroaimant et/ou un ferromagnétique.

6. Système de fixation (100) selon l'une des revendications précédentes, où le second élément magnétique (103-2) ou l'objet dentaire (101) présente une courbure de surface adaptée à une dent (105).

7. Système de fixation (100) selon l'une des revendications précédentes, où le second élément magnétique (103-2) comprend une couche déformable par la force d'attraction entre les deux éléments magnétiques (103-1, 103-2).

8. Système de fixation (100) selon l'une des revendications précédentes, où le premier élément magnétique (103-1) comprend une première surface de contact (109-1) au second élément magnétique (103-2) et/ou le second élément magnétique (103-2) comprend une deuxième surface de contact (109-2) par rapport au premier élément magnétique (103-1).

9. Système de fixation (100) selon l'une des revendications précédentes, des structures spatiales sont formées dans la première et la deuxième surface de contact (109-1, 109-2), qui provoquent une orientation prédéterminée et/ou un ajustement des liaisons par complémentarité de forme entre le premier et le second élément magnétique (103-1, 103-2).

10. Système de fixation (100) selon l'une des revendications précédentes, où l'objet dentaire (101) comprend un capteur dentaire, un composant électronique, une batterie et/ou un boîtier.

11. Système de fixation (100) selon l'une des revendications précédentes, où le premier élément magnétique (103-1), le capteur et/ou le boîtier du capteur comprennent un ou plusieurs canaux pour guider la salive vers une unité de capteur.

12. Système de fixation (100) selon l'une des revendications précédentes, où le deuxième élément magnétique (103-2) est disposé à l'intérieur de l'objet dentaire (101) ou à l'extérieur de l'objet dentaire (101) et/ou l'objet dentaire (101) comprend un boîtier qui contient un matériau ferromagnétique, ferromagnétique ou magnétique permanent ou un électroaimant.
